# FASCICULE DE BREVET EUROPEEN

(11) **EP 2 051 683 B1**
(45) Date de publication et mention de la délivrance du brevet: **28.09.2011**
(21) Numéro de dépôt: 07823373.1
(22) Date de dépôt: 31.07.2007
(51) Int. Cl.: A61J 15/00

(54) **SONDE DE GASTROSTOMIE PERCUTANEE A BALLONNET GONFLABLE ET A ANCRAGES BIODEGRADABLES**
PERKUTANE GASTROTOMIESONDE MIT AUFBLASBAREM BALLON UND BIOLOGISCH ABBAUBAREM VERANKERUNGSMITTEL
PERCUTANEOUS GASTROSTOMY PROBE WITH INFLATABLE BALLOON AND WITH BIODEGRADABLE ANCHORAGE MEANS

(30) Priorité: 02.08.2006 FR 0607074
(43) Date de publication de la demande: 29.04.2009
(73) Titulaire: Medwin France, 34240 Lamalou les Bains (FR)
(72) Inventeur: RENAUX, Serge, F-34240 Lamalou les Bains (FR)
(74) Mandataire: Gosse, Michel
(86) Numéro de dépôt international: PCT/FR2007/001319
(87) Numéro de publication internationale: WO 2008/015336

(56) Documents cités:
- WO-A-99/17708
- WO-A-03/090633
- GB-A- 2 141 346
- US-A1- 2004 097 986

## Description

### DOMAINE DE L'INVENTION

La présente invention se rapporte aux sondes de gastrostomie percutanée endoscopique (GPE) et radiologique (GPR) permettant l'accès direct à la cavité gastrique pour une alimentation entérale.

### ARRIERE PLAN TECHNOLOGIQUE

La gastrostomie percutanée est actuellement la voie de référence pour l'alimentation entérale prolongée. La simplicité et la rapidité de la technique, l'évolution du matériel font que les gastro-entérologues ou les radiologues sont de plus en plus sollicités et que la pose est accessible à tout endoscopiste ou radiologue.

Il existe deux techniques de pose :
- la technique « Pull » par voie endoscopique, principalement utilisée par les gastro-entérologues : les kits de GPE stériles comprennent généralement un trocart de ponction, un fil métallique double brin, une sonde de gastrostomie tubulaire, un moyen de rétention interne, du type collerette, une collerette de fixation externe : idéalement, une pince à corps étrangers type dent de rat ou crocodile est utilisée pour saisir le fil intragastrique, une anse diathermique ou une pince à biopsie peuvent également être utilisées ;
- la technique « Push » par la paroi abdominale, principalement utilisée par les radiologues : dans ce cas des ancres permettent d'arrimer l'estomac à la paroi abdominale le temps nécessaire à la formation d'adhérences entre la partie externe de l'estomac et la paroi abdominale, une sonde à ballonnet est alors positionnée à travers la paroi abdominale au moyen d'un dilatateur et d'une canule pelable.

Un exemple de cette technique est décrit dans le document WO 99/17708.

Les sondes sont généralement en silicone ou polyuréthane, matériaux inertes et bien tolérés. Différents calibres ou charrières sont disponibles, les sondes de petit calibre s'obstruant plus facilement.

Il existe des sondes extractibles et non extractibles.

Les sondes non extractibles, pour être remplacées, doivent être sectionnées au ras de l'orifice cutané. Le dispositif interne est ensuite poussé dans l'estomac.

Le moyen de rétention interne peut être soit récupéré par endoscopie, opération qui peut s'avérer délicate, soit évacué par les voies naturelles avec des risques d'occlusion et de perforation intestinale.

L'avantage des sondes non extractibles réside dans leur collerette interne relativement rigide résistant donc bien à une tentative d'arrachement par un patient agité.

Les sondes extractibles ont une collerette interne rétractable, ou un système de rétention dégonflable, permettant leur ablation par l'orifice cutané par traction ferme. L'avantage de ces systèmes souples est de pouvoir franchir une sténose haute, d'éviter une endoscopie mais elles ont moins de résistance à l'arrachement.

Le choix entre sondes extractibles et non extractibles en silicone ou polyuréthane dépend de l'indication, en tenant compte des avantages et inconvénients de chaque type de sonde.

Les sondes extractibles en silicone conviennent pour une nutrition entérale temporaire. Les sondes non extractibles ou en polyuréthane sont plus adaptées pour une nutrition entérale définitive ou chez un patient agité.

Le remplacement de la sonde de gastrostomie peut s'avérer nécessaire en cas d'obstruction, de détérioration du tube (fissuration, porosité, dilatation, colonisation par des candida).

La majorité des dispositifs de remplacement sont des sondes à ballonnet gonflable à l'eau, en silicone. Leur bonne adéquation avec le milieu gastrique et une collerette de rétention externe permet une bonne sécurité d'emploi.

Il existe également le bouton de gastrostomie plus court et à même la peau qui, du fait de son avantage esthétique et de son confort, est indiqué chez le sujet jeune ou ambulatoire. Il ne peut cependant que difficilement être mis en première intention et vient le plus souvent en remplacement d'une sonde déjà positionnée dans l'estomac.

La sonde selon l'invention est du type comprenant :
a) une tubulure destinée à traverser les parois stomacale et abdominale du sujet ;
b) un moyen de rétention interne, du type ballonnet gonflable, associé à des moyens d'ancrage, destiné à être solidarisé à ladite tubulure et à être maintenu plaqué contre la face interne de la paroi stomacale ;
c) une collerette externe, traversée par ladite tubulure, destinée à être plaquée contre la face externe de la paroi abdominale et à exercer, en coopération avec le moyen de rétention interne, une pression apte à plaquer la paroi stomacale contre la paroi abdominale dans la zone de la stomie.

### RESUME DE L'INVENTION

L'invention vise à réaliser une sonde du genre en question destinée à mettre en oeuvre une solution inédite et originale apte à éliminer les inconvénients susmentionnés.

Elle concerne à cet effet, une sonde de gastrostomie percutanée endoscopique d'après la revendication 1 qui se caractérise essentiellement en ce que le moyen de rétention interne est constitué d'un ballonnet gonflable, non biodégradable, associé à des moyens d'ancrage distincts maintenus par des fils, suturés en des points externes, entièrement réalisés en un polymère ou copolymère biodégradable dont la nature des polymères utilisés, leur dosage et leur masse molaire en nombre sont déterminés pour obtenir une liaison qui conserve ses propriétés mécaniques de rétention jusqu'à l'adhésion des parois stomacale et abdominale entre elles.

Le polymère ou copolymère utilisé est choisi avantageusement parmi des structures PLA et PLA GA.

Les moyens d'ancrage, qui sont constitués de petites ancres, sont durs, douloureux et traumatisants pour le sujet. D'où l'intérêt de les résorber car à cause de leur encapsulation fibreuse dans le temps, ils ne peuvent pas s'évacuer par les voies naturelles.

La biodégradation des moyens d'ancrage internes constitue l'un des avantages essentiels de l'invention en supprimant :
- les risques d'occlusion aux conséquences mortelles ;
- l'utilisation d'un endoscope qui nécessite une anesthésie ;
- les douleurs et traumatismes causés par la présence de la partie interne desdits moyens d'ancrage.

La période de cicatrisation optimale de la stomie est d'environ 21 jours. Un délai trop court conduirait à une mauvaise cicatrisation de celle-ci avec toutes les conséquences qui pourraient en résulter principalement lors de l'introduction d'une sonde de remplacement.

### PRESENTATION DES FIGURES

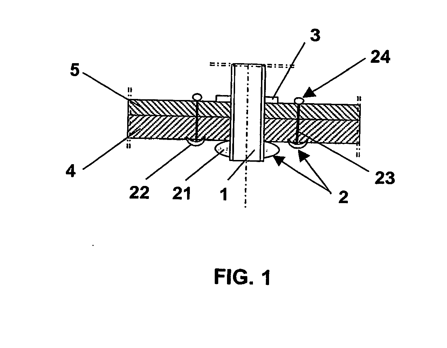

Les caractéristiques et les avantages de l'invention vont apparaître plus clairement à la lecture de la description détaillée qui suit d'au moins un mode de réalisation préféré de celle-ci donné à titre d'exemple non limitatif et représenté au dessin annexé (figure unique) qui représente, en coupe, une vue partielle d'une sonde pourvue d'une d'un ballonnet et de moyens d'ancrage, prenant en « sandwich » les parois stomacale et abdominale.

### DESCRIPTION DETAILLEE DE L'INVENTION

La sonde de gastrostomie percutanée représentée aux figures est du genre comprenant :
- une tubulure (1) destinée à traverser les parois stomacale (4) et abdominale (5) du sujet ;
- un moyen de rétention interne (2) destiné à être maintenu plaqué contre la face interne de ladite paroi stomacale (4) ;
- une collerette externe (3), traversée par la tubulure (1), destinée à être plaquée contre la face externe de ladite paroi abdominale (5) et à exercer, en coopération avec le moyen de rétention interne (2), une pression apte à plaquer la paroi stomacale (4) contre la paroi abdominale (5) dans la zone de la stomie.

Le moyen de rétention interne (2) est constitué d'un ballonnet gonflable (21), non biodégradable, associé à des moyens d'ancrage distincts (22) maintenus par des fils (23), suturés en des points externes (24), entièrement réalisés en un polymère ou copolymère biodégradable dont la nature des polymères utilisés, leur dosage et leur masse molaire en nombre sont déterminés pour obtenir une liaison qui conserve ses propriétés mécaniques de rétention jusqu'à l'adhésion des parois stomacale et abdominale entre elles.

la tubulure (1) est généralement réalisée en un matériau biocompatible non biodégradable comme par exemple en silicone ou en polyuréthane.

Le choix du polymère ou copolymère biodégradable adapté à l'application médicale considérée a fait l'objet de tests consistant à synthétiser divers polymères ou copolymères en des échantillons, immergés dans un modèle de fluide gastrique, ayant des dimensions similaires à celles des moyens de rétention internes biodégradables concernés, capables de se dégrader dans un délai bien déterminé (notamment compris entre 1 et 3 mois), ayant les caractéristiques physiques et mécaniques requises en termes d'élasticité ou de dureté, de changement de forme, de gonflement due à la prise d'eau, de décomposition...

Des tests ont été effectués sur des échantillons réalisés avec les divers polymères ou copolymères des types :
- poly acide lactique comme le PLA 50 (Mn = 21000g/mol) et le PLA 50 (Mn = 46000 g/mol) ;
- poly acide lactique-acide glycolique comme le PLA 37,5 - GA 25 (Mn = 39000 g/mol) ;
- triblocs PLA - PEG (poly éthylène glycol) - PLA comme le PLA 50 - PEG (20000) - PLA 50 (Mn = 277760 g/mol), le PLA 50 - PEG (20000) - PLA 50 (Mn = 100600 g/mol), le PLA 50 - PEG (6000) - PLA 50 (Mn = 56400 g/mol), le PLA 96 - PEG 12000
- PLA 96 (Mn = 68311 g/mol), le PLA 96 - PEG 8000 - PLA 96 (Mn = 71684 g/mol) et les PLA GA - PEG - PLA GA.

C'est dans la catégorie des polymères PLA et plus avantageusement des PLA GA que le choix s'est porté pour réaliser les moyens d'ancrage et les fils de suture où les problèmes de mémoire de forme ne se posent pas.

Des composants ayant pour effet de modifier les caractéristiques mécaniques et la vitesse de dégradation ainsi que la tolérance de l'organisme, peuvent être rajoutés auxdits copolymères

Bien entendu, l'invention n'est pas limitée aux modes de réalisation décrits et représentés pour lesquels l'homme de métier pourra prévoir d'autres variantes, en particulier dans les types de sondes utilisées et les matériaux constitutifs des sous ensembles desdites sondes à la condition que les moyens d'ancrage et les fils de suture soient entièrement biodégradables.

## Revendications

1. Sonde de gastrostomie percutanée comprenant :
a) une tubulure (1) destinée à traverser les parois stomacale (4) et abdominale (5) du sujet;
b) un moyen de rétention interne (2) destiné à être maintenu plaqué contre la face interne de ladite paroi stomacale (4) ;
c) une collerette externe (3), traversée par la tubulure (1), destinée à être plaquée contre la face externe de ladite paroi abdominale (5) et à exercer, en coopération avec le moyen de rétention interne (2), une pression apte à plaquer la paroi stomacale (4) contre la paroi abdominale (5) dans la zone de la stomie ;
**caractérisée en ce que** le moyen de rétention interne (2) est constitué d'un ballonnet gonflable (21), non biodégradable, associé à des moyens d'ancrage distincts (22) maintenus par des fils (23), suturés en des points externes (24), entièrement réalisés en un polymère ou copolymère biodégradable dont la nature des polymères utilisés, leur dosage et leur masse molaire en nombre sont déterminés pour obtenir une liaison qui conserve ses propriétés mécaniques de rétention jusqu'à l'adhésion des parois stomacale et abdominale entre elles.

2. Sonde, selon la revendication 1, **caractérisée en ce que** le polymère utilisé est du type PLA.

3. Sonde, selon la revendication 1, **caractérisée en ce que** le copolymère utilisé est du type PLA GA.

## Claims

1. Percutaneous stomach probe including:
a) a tubing (1) intended to pass through the stomach (4) and abdominal (5) walls of the patient;
b) an internal retaining means (2) intended to be maintened pressed against the inner surface of said stomach wall (4);
c) an external flange (3), traversed by the tubing (1), intended to be pressed against the outer surface of said abdominal wall (5) and, in cooperation with the internal retaining means (2), to exert pressure adapted to press the stomach wall (4) against the abdominal wall (5) in the region of the stomach; **characterised in that** the internal retaining means (2) consists of a non-biodegradable inflatable balloon (21) combined with separate anchoring means (22) held by wires (23) sutured at external points (24) and made entirely of a biodegradable polymer or copolymer of which the nature of the polymers used, their dosage and their molar mass thereof by number are determined so as to obtain a connection which preserves the mechanical retention properties of same until the stomach and abdominal walls are adhered to one another.

2. Probe according to claim 1, **characterised in that** the polymer used is of the PLA type.

3. Probe according to claim 1, **characterised in that** the copolymer used is of the PLGA type.

## Patentansprüche

1. Perkutane Gastrostomiesonde, umfassend:
a) einen Schlauch (1), der dazu vorgesehen ist, die Magen- (4) und Bauchwand (5) des Patienten zu durchqueren;
b) ein internes Rückhaltemittel (2), das dazu vorgesehen ist, gegen die innere Seite der Magenwand (4) gedrückt gehalten zu werden;
c) einen externen Kragen (3), der von dem Schlauch (1) durchquert wird, der dazu vorgesehen ist, gegen die äußere Seite der Bauchwand (5) gedrückt zu werden und in Zusammenarbeit mit dem internen Rückhaltmittel (2) einen Druck auszuüben, der dazu geeignet ist, die Magenwand (4) gegen die Bauchwand (5) im Bereich der Ostomie zu drücken;
**dadurch gekennzeichnet, dass** das interne Rückhaltemittel (2) aus einem aufblasbaren, nicht biologisch abbaubaren Ballon (21) besteht, der mit verschiedenen Verankerungsmitteln (22) verbunden ist, die von Fäden (23) gehalten werden, die an externen Punkten (24) vernäht sind, die vollständig aus einem biologisch abbaubaren Polymer oder Copolymer durchgeführt sind, wobei die Art der verwendeten Polymere, ihre Dosierung und die Zahl ihrer Molmasse festgelegt werden, um eine Verbindung zu erhalten, die ihre mechanischen Eigenschaften des Rückhalts bis zur Adhäsion der Magenwand und der Bauchwand untereinander beibehält.

2. Sonde nach Anspruch 1, **dadurch gekennzeichnet, dass** das verwendete Polymer des Typs PLA ist.

3. Sonde nach Anspruch 1, **dadurch gekennzeichnet, dass** das verwendete Copolymer des Typs PLA GA ist.
